# EUROPEAN PATENT SPECIFICATION

(11) **EP 1 037 577 B1**
(45) Date of publication and mention of the grant of the patent: **19.09.2001**
(21) Application number: 98924499.1
(22) Date of filing: 17.06.1998
(51) Int. Cl.: A61F 2/44

(54) **METALLIC INTERVERTEBRAL DISC**
METALLISCHE ZWISCHENWIRBELSCHEIBE
DISQUE INTERVERTEBRAL METALLIQUE

(30) Priority: 20.06.1997 GR 97100248
(43) Date of publication of application: 27.09.2000
(73) Proprietor: Tsitsopoulos, Philip, 546 23 Thessaloniki (GR)
(72) Inventor: Tsitsopoulos, Philip, 546 23 Thessaloniki (GR)
(74) Representative: Becker Kurig Straus
(86) International application number: GR9800017
(87) International publication number: WO9858604

(56) References cited:
- WO-A-96/40014
- WO-A-97/32547
- DE-A- 19 519 101
- FR-A- 2 703 580
- FR-A- 2 724 312
- FR-A- 2 733 413
- US-A- 5 192 327

## Description

### Technical field:

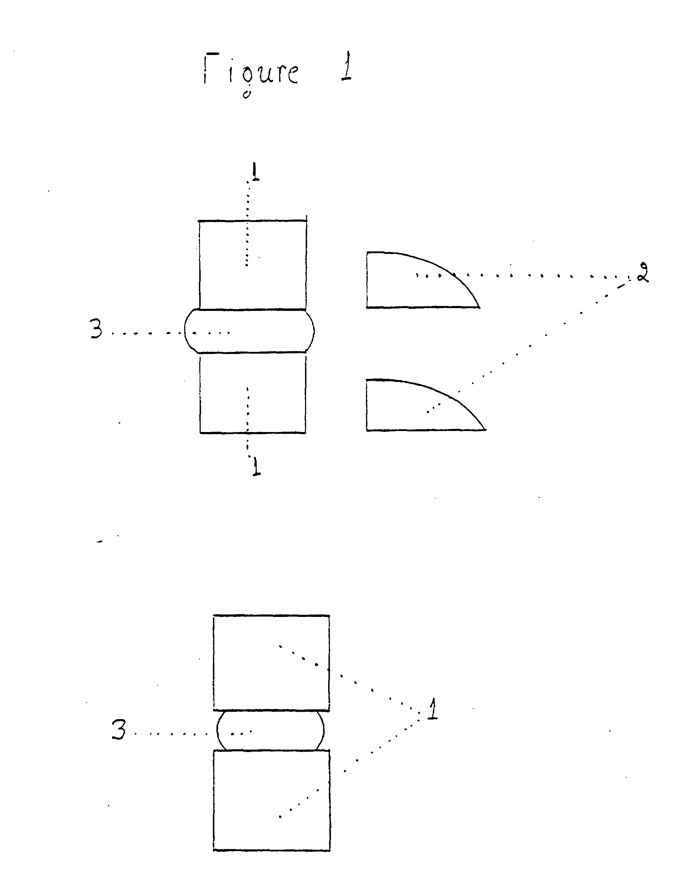

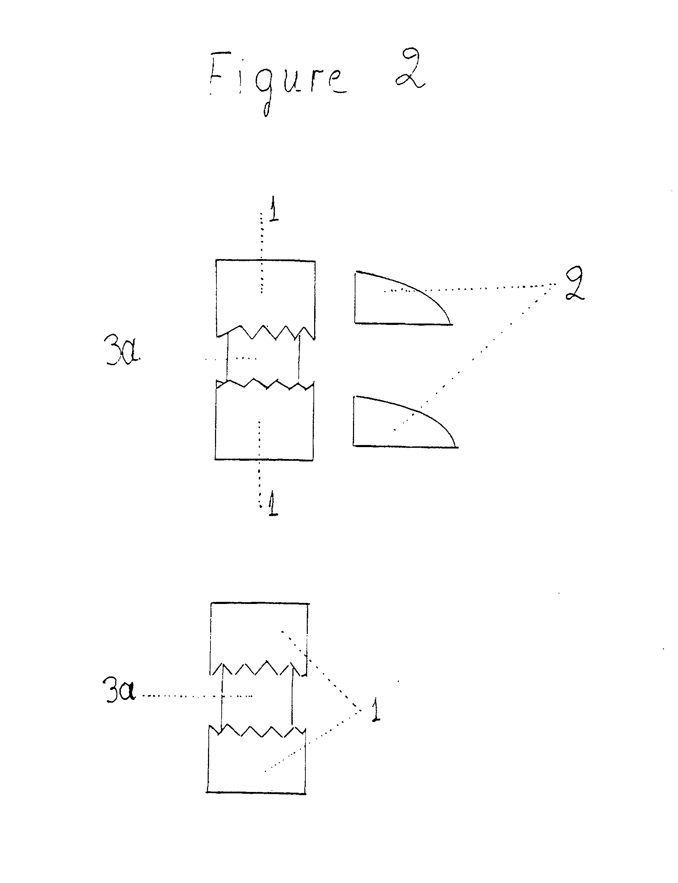

A metallic dentate ring prosthesis, (Figures 1,2) filling the gap between two cervical vertebrae, after removal of the intervertebral disc, for use in surgical decompression and fusion-stabilization of the cervical spine.

Vertebral body=1, vertebral laminae=2. normal intervertebral disc=3. substitute intervertebral disc-prosthesis=3a

Ring, substitute, prosthesis, implant. are terms to be used.

### Evaluation of the existing relative status of knowledge

Their main purpose after filling the gap between two cervical vertebrae following the removal of the intervertebral disc is to correct and maintain an adequate intervertebral space in order to allow the spinal roots to course freely through the intervertebral foramina. One other important purpose is to stabilize the vertebral column immobilizing the joint between the vertebral bodies and to encourage osteogenesis.

Some of other prostheses allow unrestricted motion in use in the replacement of spinal discs segments.

The prostheses in use today come in different shapes, dimensions and finishing. Two main characteristics are that the thickness of their ring is less than 1mm, and that other prostheses have ring and edges of the ring with the same thickness thus allowing the edges to penetrate the vertebral bodies. This results to loss of height of the intervertebral space and to compression of the spinal roots which run through the adjacent intervertebral foramina.

The application of loading shearing forces in some prostheses with many dents-serrated structure, i.e. more than 15 up to 20 dents, may act as the function of the saw namely as the saw is sawing the wood, resulting in destroying the in-between the dents holding osseous mass, which can lead to loosening of the stable positioning of the implant.

Other used prostheses without dents, although they have built-in mechanism to maintain the height of the intervertebral space, however they carry the risk of migration, and they cannot be applied securely in more than one invertebral spaces without further stalilization techniques with plates and screws. Moreover this additional procedure increases considerably the surgical time and the risk of contamination.

Other artificial discs consisting of two components but being mobile (moveable) they allow unrestricted rotational and flexion-extension bending motion in the spinal disc segments they replace, thus, their function is exactly the opposite of what is needed in fusion-stabilization spine procedures.

In addition, those of the existing metallic rings without dents require major instrumentation, time consuming insertion techniques and additional stabilization materials (plates and screws) in cases of insertion of prosthesis in more than two inlervertebral spaces.

### The advantages of our prosthesis are:

a) Because of the special dents. it is stabilized in situ securely minimizing the risk of migration to any direction.
b) Encourages osteogenesis in the inner opening of the ring.
c) Maintains adequate intervertebral height due to its special stops between the dents controlling, thus, absolutely the degree of the impaction and the height of the intervertebral space preserving a quite enough holding osseous mass, in-between the dents.
d) Because the thickness of the ring-cylinder and the thickness of the dents is different, the implant is secured between the vertebrae preventing the penetration furthermore into the vertebral body and the subsequent loss of the intervertebral space height with all its related consequences.
e) Because of its special design it penetrates only at a controllable predetermined depth and succeeds in preventing further penetration.
f) Requires much less instrumentation and surgical time for insertion, minimizing thus, the risk of contamination.

Obviously, even in cases where two or more prostheses are used there is no need for further stabilization with plates and screws.

### REVEALING THE INVENTION

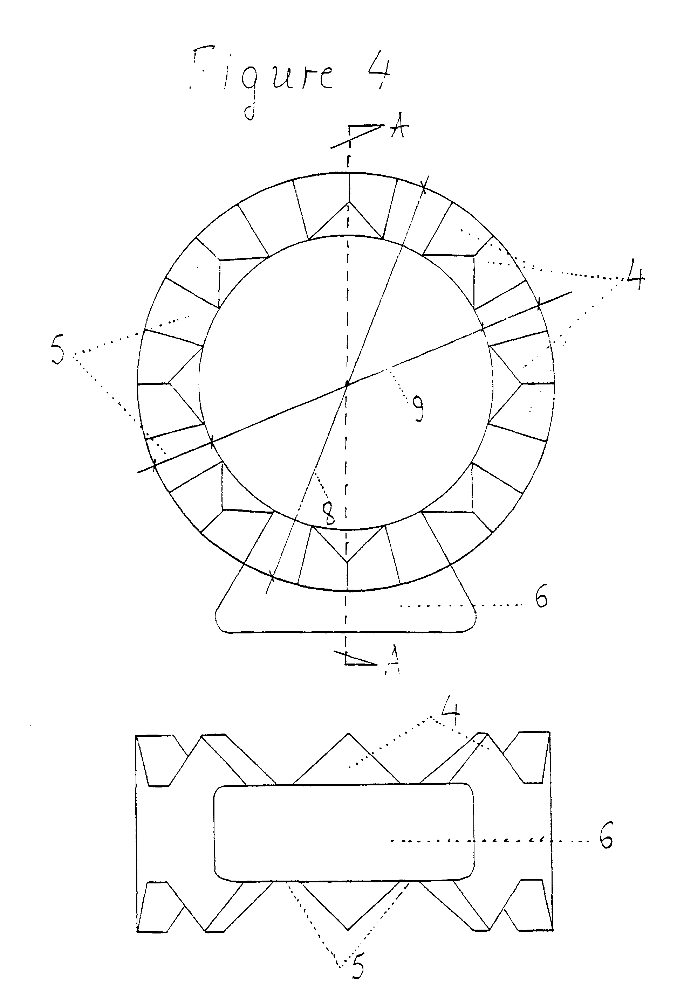

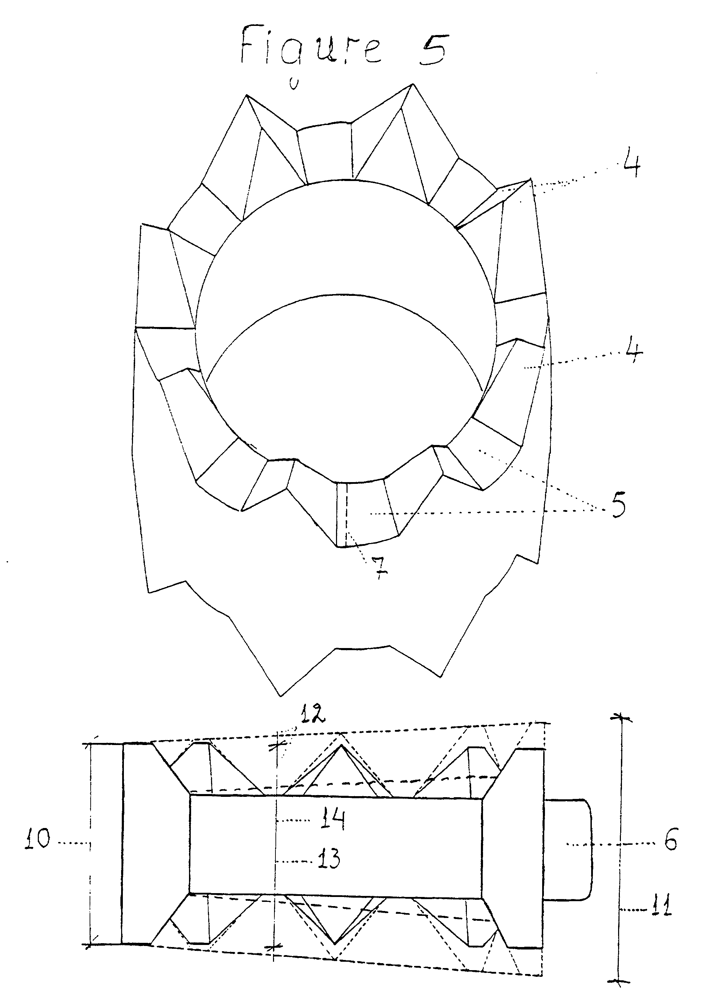

Metallic intervertebral disc (3a) with an outer periphery less than the periphery of the vertebral body so that the prosthesis interposes in-between the cervical vertebral bodies after removal of the intervertebral disc (Figure 2) This prosthesis is a cylinder with the same thickness all over. parallel or trapezium (in profile). Its periphery could be circle or elliptic (oval) (Figures 4, 5) This metallic intervertebral disc (3a) in its upper and lower surface and all round the cylinder finishes with dents (4) and footsteps (5) stops (sitting positions) (Figures 2, 4, 5).

This metallic intervertebral disc in one part of the outer periphery of the cylinder (this part then is the anterior part of the prosthesis) finishes in a holder (6) which facilitates the positioning of the metallic disc during its application in-between the two vertebral bodies (Figures 4, 5), viewed from below, view, A-A section). In the trapezoid prosthesis the holder (6) is the anterior (the higher - 7,5mm) part of the outer periphery of the cylinder [Figures 4, 5, A-A section].

The thickness (7) of the ring of the prosthesis is 2mm, the external (8) ring diameter 14mm and the internal (9) ring diameter 10mm. The height of the prosthesis (dents included) parallel (10) or trapezium (11)] is 6mm to 7,5mm. Dent height (12) is 1,5mm - 2mm. The height of the prosthesis without the dents is 3mm - 4,5mm [(parallel (13) or trapezium (14)]. (Figures 4, 5).

The declivity or slope of the dent (15) is 2mm - 3mm, the distance edge dent lengthring (16) is 1,5mm or 2mm. The thickness of the dent at the edge dent (which edge is sharp)- edge dent length (17) is 1mm and the internal dent angle (18) is 45° - 90°

The thickness of each dent gets progressively less going upwards i.e. from 2mm at the base of the dent- thickness of the ring - cylinder (7) to 1mm at the edge dent - edge dent length (17).

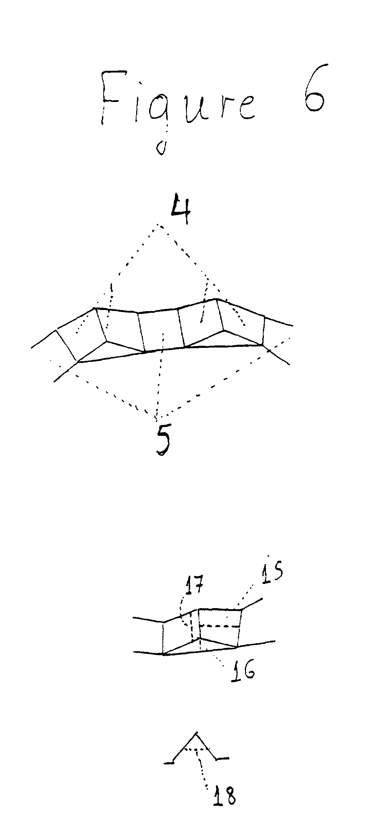

The dent gets thinner - narrow- at its edge, at the expense of its inner side (part), preserving its outer side (part) at the same vertical level with the outer periphery of the ring-cylinder. (Figures 4, 5, 6).

In relation to the level of the cervical spine where the prosthesis will be used, the number of the dents will vary accordingly, usually 8 or less all round the upper and lower surface of the cylinder. (Figures 4, 5).

It is obvious (because of the normal lordosis of the cervical spine when it is preserved) that in the case of the trapezoid prosthesis the higher dents (2mm) and therefore the higher part of the cylinder (7.5mm), when applied in-between the bodies of the vertebral bodies, have to be positioned on the anterior part of the intervertebral space. Thus, the anatomy and the function of the spine (cervical in particular) is maintained even more.

The shape of the holder (6) is a small cylinder with horizontal the anterior surface and concave the posterior surface where it continues transitioned into the (principal) cylinder of the prosthesis. The distance, where the horizontal surface of the holder (anterior surface) changes having a perpendicular course to the cylinder of the prosthesis, is 2mm (i.e. thickness of the holder sideways).

The holder (6) of the prosthesis is shaped in such a manner to hold the prosthesis with safety and to easily remove it, if the prosthesis is not applied correctly.

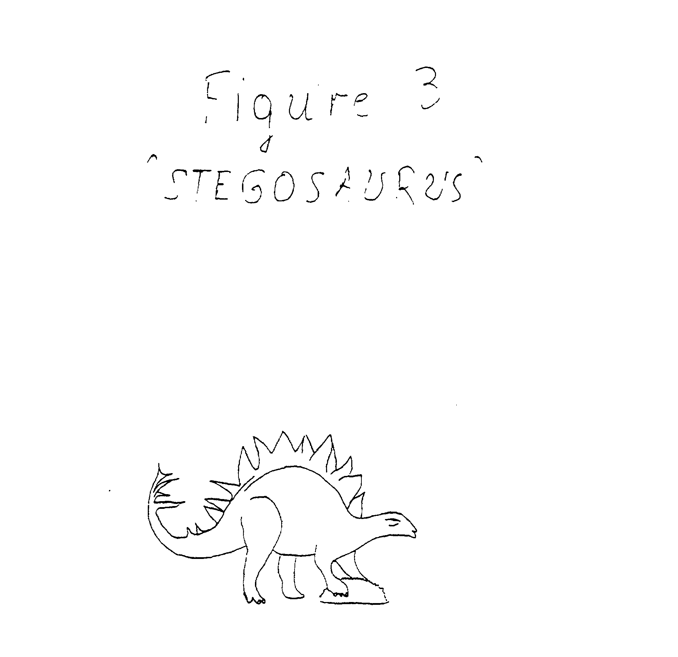

Thus, our intervertebral disc has the following characteristics:
1. It restores the height of the intervertebral space back to normal (i.e. to 1/3 of the vertebral body).
2. It has **α**. such dents (4) to inhibit the migration of the artificial disc β, in-between the dents has sitting positions (stops) (5) which inhibit the impaction of the artificial disc and c.it has an internal opening which is filled with autologous osseous implant so that a bony bridge (osteogenesis) takes places between the upper and lower lying vertebrae.
3. It has different thickness cylinder (ring) and dents to avoid further penetration into the adjacent vertebrae.
4. It stabilizes the joint between the vertebrae immediately from the first postoperative day and secondary the bony bridge takes place after 6 months time. This prosthesis differs from the others because its dents succeed one each other in such a manner so that the existence of a stop (like footstep) in-between the dents inhibits the complete impaction of the prosthesis in the vertebral bodies. It is nature, the structure and the shape of the special dents (4) (like stegosaurus back) (Figure 3) combined with the in- betwcen stops (5) and the difference in thickness between cylinder -ring (7) and dents (17), (Figures 5, 6) which account not only for a better impaction but also for a more secure application and a better holding of this prosthesis after the removal of the intervertebral disc.

The better impaction of this prosthesis is achieved because of the dents whose special shape getting progressively narrow upwards (edge dent length) and having a proper finishing, (sharp edge), which is not like a needle, pierce into the vertebral bodies without penetrating the bodies circumferentially.

The secure application and better holding of this prosthesis is due to the in-between the dents stops, which inhibit the prosthesis to get further impacted, so that in combination with the dents and the succession dents - stops with the special characteristics they possess (shape and dimensions) they do not allow the migration of the prosthesis.

Additionally, the different thickness between cylinder (ring=2mm) and dents (1mm) accounts for preventing further penetration into the vertebrae.

Because of its special design (Figures 4, 5) this prosthesis succeeds in both the following purposes: α to penetrate controllably predetermined up to the point we desire, into the adjacent upper and lower terminal plates of the vertebral bodies and β. to stop to a secure point in the vertebral bodies which is not the case with other prostheses.

This prosthesis secures from migration more than any other prosthesis.

It does not require built-in mechanism for proper placement, on the other hand only a limited number of instruments are required for its application constituting its insertion technique very simple.

Obviously, even in cases with more than two prostheses applied i.e. in two or more cervical intervertebral spaces there is no need for further stabilization with plates and screws.

To obtain experience in applying this prosthesis is easy, takes less time of training and can be easily achieved by neurosurgeons or orthopaedic surgeons, assuring a good final result.

This invention is compatible with less invasive surgical techniques:
its cost combined with the cost of its application is low because:
   1. It is simple in construction
   2. There is no need of any special application instrument
   3. Its use avoids the additional placement of stabilizing -fusion plates and screws in cases of multiple prostheses.
   4. The method of positioning the prosthesis is less tiring for the surgeon and the whole procedure is more functional for the patient.

### Example of an application of this invention

Male 46 years suffers from intractable pain radiating to the right arm for six months after a car accident. Clinically motor power diminished in the segments served by the cervical roots C₅,C₆. The neurophysiological and neuroradiological investigation in combination with the clinical examination revealed a C₅,C₆ (cervical) intervertebral disc prolapse compressing severely the relevant roots.

The patient in order to be cured must have an operation. This operation consists of a cervical discectomy that is removal of the diseased prolapsed intervertebral disc at the level C₅,C₆ which compresses the neural elements. However, after the removal of the intervertebral disc the height of the intervertebral space diminishes and this may result in roots compression.

To avoid this situation the intervertebral space must be filled. Thus, the metallic intervertebral disc interposes in-between the two vertebral bodies restores the anatomy of the area keeping the adjacent neural elements free and leaving undisturbed the course of the roots. A major problem which can occur after the application of the prosthesis is the migration of the prosthesis.

It is because of the special dents (and, in certain number 8 or less) and the sitting positions (stops) succeeding one each other and having this special shape and dimensions, in addition to the difference in thickness between ring (cylinder) and dents that this metallic intervertebral disc holds its position stable once fitted and cannot migrate after its positioning.

Specifically, it is emphasized that in our prosthesis the internal bigger (wider) opening (compared to the opening of other prostheses which is smaller) helps more in osteogenesis and therefore accelerates the production of the bony bridge (which is in our case bigger in volume) between the two bodies.

## Claims

1. Metallic intervertebral disc which consists of one single piece of a metallic titanium alloy ring cylinder with an outer periphery less than the periphery of the vertebral body so that the prosthesis interposes in between the cervical vertebral bodies after the removal of the intervertebral disc; on the upper and on the lower surface and all around the ring cylinder of the substitute, are teeth similar in shape with such length and finishing to penetrate upwards and downwards into the vertebral bodies, the sharpness of the teeth is defined by the thickness of the base of the tooth which is 2 mm and the thickness at the point of the tooth is 1 mm, while the tooth height is 1,5-2 mm, the difference between prosthesis cylinder thickness which is 2 mm and tooth point thickness which is 1 mm, does not allow further penetration keeping the rest cylinder in the intervertebral space and outside the vertebral bodies; the number of the teeth is 8 or fewer in each of the upper and lower surface of the implant; in-between the teeth are interposed sitting positions acting as stops, which are defined by the distance between adjacent teeth which is 4 mm at the internal - 10 mm - ring diameter and 5,4 mm at the external - 14 mm - ring diameter, the difference between prosthesis cylinder thickness and tooth thickness and the interposed sitting positions prevent migration and further penetration of the cylinder into the adjcent vertebral bodies once the implant is placed, so that the desired height of the intervertebral space is maintained, said 8 or fewer teeth allow enough osseous mass between them to avoid a sawing effect as observed in the presence of multiple teeth under a shearing force.

2. Metallic intervertebral disc as it is referred to in claim 1 having a height without the teeth of 3 - 4,5 mm like the height of the normal intervertebral disc.

3. Metallic intervertebral disc as it is referred to in claims 1 to 2, in which the cylinder has the same thickness throughout, or has a trapezoidal profile and a circular or elliptical or oval periphery.

4. Metallic intervertebral disc as it is referred to in claims 1 to 3, which has a height bigger than the height of the intervertebral disc to cover cases of partial replacement of the underlying or the overlying vertebral bodies.

## Patentansprüche

1. Metallische Zwischenwirbelscheibe, die aus einem einzelnen Stück eines Ringzylinders aus einer metallischen Titanlegierung besteht, mit einem äußeren Rand, kleiner als der Rand des Wirbelkörpers, so dass die Prothese zwischen den Wirbelsäulenwirbelkörpern nach der Entfernung der Bandscheibe zwischenliegt; auf der oberen und auf der unteren Oberfläche und um den gesamten Ringzylinder des Ersatzes befinden sich Zähne ähnlicher Form mit einer solchen Länge und Oberflächenbeschaffenheit, um aufwärts und abwärts in die Wirbelkörper einzudringen, wobei die Schärfe der Zähne definiert ist durch die Dicke der Basis des Zahns, die 2 mm beträgt, und durch die Dicke an der Spitze des Zahns, die 1mm beträgt, während die Zahnhöhe 1,5 bis 2 mm beträgt, wobei der Unterschied zwischen der Dicke des Prothesenzylinders, die 2 mm beträgt, und der Zahnspitzendicke, die 1 mm beträgt, kein weiteres Eindringen erlaubt, und den Restzylinder in dem Zwischenwirbelraum außerhalb der Wirbelkörper hält; wobei die Anzahl der Zähne in jeder oberen und unteren Oberfläche des Implantats 8 oder weniger beträgt; wobei sich zwischen den Zähnen zwischenliegende Sitzstellen befinden, die als Stopps dienen, die durch den Abstand zwischen benachbarten Zähnen, der an dem inneren 10 mm Ringdurchmesser 4 mm, und am äußeren 14 mm Ringdurchmesser 5,4 mm beträgt, wobei der Unterschied zwischen der Prothesenzylinderdicke und der Zahndicke und den zwischenliegenden Sitzstellen ein Wandern und weiteres Eindringen des Zylinders in die benachbarten Wirbelkörper verhindert, wenn das Implantat eingesetzt ist, so dass die gewünschte Höhe des Zwischenwirbelraums aufrecht erhalten wird, wobei die 8 oder weniger Zähne genügend Knochenmasse zwischen ihnen erlauben, um einen Sägeeffekt, wie in der Gegenwart von mehreren Zähnen unter einer Scherkraft beobachtet, zu vermeiden.

2. Metallische Zwischenwirbelscheibe gemäß Anspruch 1, die eine Höhe ohne die Zähne von 3 bis 4,5 mm wie die Höhe der normalen Bandscheibe aufweist.

3. Metallische Zwischenwirbelscheibe gemäß Anspruch 1 oder 2, wobei der Zylinder entweder überall die selbe Dicke oder ein trapezförmiges Profil und einen kreisförmigen, elliptischen oder ovalen Rand aufweist.

4. Metallische Zwischenwirbelscheibe, gemäß einem der Ansprüche 1 bis 3, die eine Höhe aufweist, die größer als die Höhe der Bandscheibe ist, um Fälle teilweisen Ersatzes der darüber- oder darunterliegenden Wirbelkörper abzudecken.

## Revendications

1. Disque intervertébral métallique qui consiste en une seule pièce d'un cylindre annulaire en alliage métallique de titane qui présente un périmètre extérieur inférieur au périmètre du corps de vertèbre de telle façon que la prothèse s'intercale entre les corps de vertèbres cervicales après l'enlèvement du disque intervertébral; il y a sur la surface supérieure et sur la surface inférieure et tout autour du cylindre annulaire du substitut des dents semblables en forme et d'une longueur et d'une finition telles qu'elles pénètrent vers le haut et vers le bas dans les corps de vertèbres, le pouvoir de pénétration des dents est défini par l'épaisseur de la base de la dent qui est de 2 mm et l'épaisseur à la pointe de la dent qui est de 1 mm, tandis que la hauteur de la dent est de 1,5-2 mm, la différence entre l'épaisseur de cylindre de la prothèse qui est de 2 mm et l'épaisseur de la pointe de dent qui est de 1 mm ne permet pas de pénétration supplémentaire laissant le cylindre restant dans l'espace intervertébral et à l'extérieur des corps de vertèbres; le nombre des dents est de 8 ou moins dans chacune des surfaces supérieure et inférieure de l'implant; entre les dents sont interposés des emplacements d'assise faisant office d'arrêts qui sont définis par la distance entre des dents adjacentes qui est de 4 mm au diamètre d'anneau intérieur - 10 mm - et de 5,4 mm au diamètre d'anneau extérieur - 14 mm -, la différence entre l'épaisseur du cylindre de prothèse et l'épaisseur de dent et les emplacements d'assise interposés empêche la migration et la pénétration supplémentaire du cylindre dans des corps de vertèbres adjacents une fois l'implant mis en place, de telle façon que la hauteur désirée de l'espace intervertébral soi conservée, lesdites 8 dents ou moins laissent suffisamment de masse osseuse entre elles pour éviter un effet de sciage comme on l'a observé en présence de multiples dents sous l'effet d'une force de cisaillement.

2. Disque intervertébral métallique selon la revendication 1 présentant une hauteur sans les dents de 3 - 4,5 mm similaire à la hauteur du disque intervertébral normal.

3. Disque intervertébral métallique selon les revendications 1 à 2 dans lequel le cylindre a la même épaisseur partout ou a un profil trapézoïdal et un périmètre circulaire ou elliptique ou ovale.

4. Disque intervertébral métallique selon les revendications 1 à 3 qui présente une hauteur plus grande que la hauteur du disque intervertébral pour pouvoir être utilisé dans les cas d'un remplacement partiel des corps de vertèbres inférieur ou supérieur.
